# EUROPÄISCHE PATENTSCHRIFT

(11) **EP 1 654 405 B1**
(45) Veröffentlichungstag und Bekanntmachung des Hinweises auf die Patenterteilung: **15.10.2008**
(21) Anmeldenummer: 04763849.9
(22) Anmeldetag: 06.08.2004
(51) Int. Cl.: C30B 7/00, B01J 19/00, G01N 33/68

(54) **VERFAHREN ZUM BEHANDELN EINES KRISTALLS DURCH AUFBRINGEN VON MIKROTROPFEN AUF DEN KRISTALL**
METHOD FOR TREATING A CRYSTAL BY APPLYING MICRODROPS THERETO
PROCEDE POUR TRAITER UN CRISTAL PAR APPLICATION DE MICROGOUTTES SUR LE CRISTAL

(30) Priorität: 06.08.2003 DE 10336110
(43) Veröffentlichungstag der Anmeldung: 10.05.2006
(73) Patentinhaber: Proteros Biostructures GmbH, 82152 Martinsried (DE)
(72) Erfinder: NEUEFEIND, Torsten, 82131 Gauting (DE); BRANDSTETTER, Hans, 82131 Gauting (DE); KIEFERSAUER, Reiner, 82061 Neuried (DE)
(74) Vertreter: Huenges, Martin
(86) Internationale Anmeldenummer: PCT/EP2004/008812
(87) Internationale Veröffentlichungsnummer: WO 2005/017236

(56) Entgegenhaltungen:
- EP-A- 0 278 131
- WO-A-99/45379
- DE-C- 19 842 797
- US-A1- 2002 153 055
- US-A1- 2002 191 048
- US-A1- 2003 049 642
- STEWART P S ET AL: "A comparison of microbatch and vapour diffusion for initial screening of crystallization conditions" JOURNAL OF CRYSTAL GROWTH, NORTH-HOLLAND PUBLISHING CO. AMSTERDAM, NL, Bd. 168, Nr. 1, 1. Oktober 1996 (1996-10-01), Seiten 170-174, XP004013784 ISSN: 0022-0248
- CLEMONS W M ET AL: "Crystal structure of the 30 S ribosomal subunit from Thermus thermophilus: purification, crystallization and structure determination" JOURNAL OF MOLECULAR BIOLOGY, LONDON, GB, Bd. 310, Nr. 4, 20. Juli 2001 (2001-07-20), Seiten 827-843, XP004480481 ISSN: 0022-2836
- CHAYEN N E ET AL: "Protein crystallization for genomics: towards high-throughput optimization techniques" ACTA CRYSTALLOGRAPHICA, SECTION D (BIOLOGICAL CRYSTALLOGRAPHY) MUNKSGAARD INTERNATIONAL BOOKSELLERS AND PUBLISHERS FOR INT. UNION CRYSTALLOGR DENMARK, Bd. D58, 2002, Seiten 921-927, XP009013224 ISSN: 0907-4449
- NIENABER V L ET AL: "Discovering novel ligands for macromolecules using X-ray crystallographic screening" NATURE BIOTECHNOLOGY, NATURE PUBLISHING, US, Bd. 18, Nr. 10, Oktober 2000 (2000-10), Seiten 1105-1108, XP002250092 ISSN: 1087-0156
- KIEFERSAUER R. ET AL: 'Protein-Crystal Density by Volume Measurement and Amino-Acid Analysis' J. APPL. CRYST. Bd. 29, 1996, Seiten 311 - 317 ISSN: 0021-8898
- KIEFERSAUER R. ET AL: 'A novel free-mounting system for protein crystals: transformation and improvement of diffraction power by accurately controlled humidity changes' J. APPL. CRYST. Bd. 33, 2000, Seiten 1223 - 1230 ISSN: 0021-8898
- SJÖGREN T. ET AL: 'Protein crystallography in a vapour stream: data collection, reaction initiation and intermediate trapping in naked hydrated protein crystals' J. APPL. CRYST. Bd. 35, 2002, Seiten 113 - 116 ISSN: 0021-8898
- RÖSSLE M.: 'Fast Intracrystalline Hydration of beta-Chitin Revealed by Combined Microdrop Generation and On-Line Synchrotron Radiation Microdiffraction"' BIOMACROMOLECULES Bd. 4, 04 November 2003, Seiten 981 - 986
- GLOWACKI B.A.: 'Preparation of Bi2Sr2CaCu2O8-x tracks and thick films by jet printing' SUPERCOND. SCI. TECHNOL. Bd. 13, 2000, Seiten 584 - 591

## Beschreibung

Die vorliegende Erfindung bezieht sich auf ein Verfahren zum Behandeln eines Kristalls mit einer Flüssigkeit und insbesondere auf ein Verfahren zum Einbringen von Liganden und/oder Inhibitoren in eine Proteinkristallstruktur.

In der Proteinkristallographie kommt es häufig vor, daß vor der kristallographischen Messung Liganden oder Inhibitoren in eine Proteinstruktur bzw. einen Proteinkristall eingebracht werden sollen. Ziel ist es dabei, die kristallographische Struktur eines Proteins ohne und mit Ligand oder Inhibitor zu vergleichen und die räumliche Anordnung des Liganden oder Proteins zu ermitteln. Liganden können hierbei alle an eine Protein oder Polypeptid bindenden Moleküle oder Substanzen sein, die bspw. inhibitorische Wirkung oder auch agonistische Wirkung auf die Funktion des Proteins haben können. Ggf. können Liganden organisch-chemische Moleküle sein oder auch (modifizierte) Antikörper oder Antikörperfragmente, native Bindungspartner oder Fragmente, ggf. modifiziert, vom kristallisierten Protein. Weiterhin werden regelmäßig auch Schwermetallatom-Derivate in der Kristallographie benötigt, um die entsprechende Phaseninformationen zu erhalten. Ein Ligand i.S. der vorliegenden Erfindung kann daher auch ein an das kristallisierte Protein bindendes Schwermetallatom(salz)

Ein im Stand der Technik bekanntes Verfahren zum Einbringen von Liganden, bspw. Inhibitoren, ist das sogenannte "Soaking" mit einem Puffer, der aus der Kristallisationslösung sowie dem Liganden besteht. Falls ein in den Kristall zu "soakender" Ligand schlecht oder nur schwerlöslich sind, können dem Puffer zur Erhöhung der Löslichkeit desselben weitere Substanzen als Löslichkeitsverbesserer zugesetzt werden. Beispielsweise kann es sich um Lösungsmittel wie DMSO (Dimethylsulfoxid), TFE, Ethanol, 2-Nitropropan oder andere organische Lösungsmittel, insbesondere chlorierte Lösungsmittel, ggf. auch Emulgatoren handeln.

Das "Soaking"-Verfahren besitzt verschiedene Nachteile. So besteht ein Nachteil darin, daß die Kristalle beim "Soaking" einer anderen Umgebung ausgesetzt werden müssen, wodurch der Kristall Schaden erleiden kann, d.h. insbesondere, dass die Mikrostruktur des Kristalls nach dem "Soaking" Unregelmäßigkeiten aufweist, die das Diffraktionsvermögen des Kristalls beeinträchtigen. Sollen z.B. schwerlösliche Inhibitoren oder Liganden in die Proteinkristallstruktur eingebracht werden, so benötigt man sehr hohe Konzentrationen an Lösungsmittel. Gerade hohe Lösungsmittelkonzentrationen führen aber häufig zur Zerstörung der fragilen Proteinkristalle, wie zuvor erwähnt.

Der Artikel "Protein crystallography in a vapour stream: data collection, reaction initiation and intermediate trapping in naked hydrated protein crystals" in J. Appl. Cryst. (2002), vol.35, S.113-116 von T. Sjörgren et al. beschreibt ein Verfahren zu Experimenten an nackten, ungefrorenen Proteinkristallen. Der Kristall wird in einem dünnen Film seiner Mutterlösung in einem konventionellen Cryoloop montiert und in einem nassen Dampfstrom platziert, dessen Zusammensetzung und Temperatur geregelt werden kann. Reaktionen können in vielen Proteinkristallen initiiert werden, indem die Zusammensetzung des Gasstroms geändert wird, um eine neue chemische Umgebung um den Kristall herum zu schaffen und Substrate/Reaktanden entweder in der Gasphase oder als Mikrotropfen zuzuführen. Reaktionen können in dem Kristall ausgelöst werden durch Mikroinjektion von Substraten in die den Kristall umgebende Mutterlösung, wenn dieser im Cryo-loop befestigt ist.

Der Artikel "Fast intracrystalline Hydration of beta-chitin Revealed by Combined Microdrop Generation and On-Line Synchrotron Radiation Microdiffraction" in Biomacromolecules 2003, 4, S.981-986 von M. Rössle et al. offenbart gemäss Einleitung ein Verfahren zum Untersuchen der Kinetik von Mikrostrukturänderungen, die von flüssigen Mikrotropfen an Festkörpern hervorgerufen werden. Als Demonstrationsbeispiel wird eine Verfahren zum Behandeln von hochkristallinem beta-chitin mit den folgenden Schritten beschrieben:
- Befestigen der hochkristallinen beta-chitin Probe mittels Klebstoff an einer Glasspitze, die ihrerseits an einem Standard Probenhalter (Hampton Research) angebracht wird,
- Trocknen der Probe für einige Stunden in einem Vakuumofen bei 50°C,
- Transfer der Probe zum Mikrogoniometer der European Synchrontron Radiation Facility und Ausrichten der Probe am Mikrogoniometer,
- direktes Aufbringen von Mikrotropfen von destilliertem Wasser auf die Probe.

Der Artikel "Preparation of Bi2Sr2CaCu2O8-x tracks and thick films by jet printing" von B.A.Glowacki in "Supercond. Sci. Technol.", vol.13 (2000), S.584-591 offenbart ein Verfahren zum Erzeugen von Dickfilmbahnen eines Supraleiters, indem eine geeignete Lösung durch Tintenstrahldrucken mittels eines "droplet on request"-systems auf einen MgO-Einkristall aufgebracht wird.

Darüber hinaus besteht ein weiterer Nachteil des herkömmlichen "Soaking"-Verfahrens in dem hohen Zeitaufwand des Verfahrens. Dieser ist zum einen durch die unter Umständen zahlreichen (repetitiven) "Soaking"-Prozesse bedingt, die, ggf. unter Veränderung der Konzentrationsverhältnisse des zu "soakenden" Liganden durchlaufen werden müssen, um überhaupt eine geeignete, die Liganden oder Inhibitoren enthaltende, also komplexierte Proteinkristallstruktur (Cokristall) zu erhalten, und zum anderen dadurch, daß bereits ein einzelner Soaking-Prozeß bereits sehr zeitaufwendig sein kann, da bspw. die Diffusionskinetik beachtet werden muß.

Ein weiterer Nachteil des "Soaking"-Verfahrens besteht darin, daß röntgenkristallographische Untersuchungen oder Untersuchungen des Proteinkristalls mit Synchrotronstrahlung während des "Soaking"-Verfahrens technisch nicht möglich sind.

Die Aufgabe der Erfindung besteht nun darin, ein Verfahren zur Behandlung eines Kristalls mit einer Substanz zu schaffen, die im Vergleich zu bisherigen Verfahren unter anderem eine schonendere Behandlung von Kristallen und insbesondere Proteinkristallen und die einfachere und/oder effizientere Herstellung von komplexierten Kristallen und insbesondere Proteinkristallen sowie die einfache Herstellung von bisher nur schwer oder überhaupt nicht herstellbaren komplexierten Kristallen und insbesondere Proteinkristallen erlauben.

Diese Aufgabe wird durch ein Verfahren zum Behandeln eines Kristalls mit einer Flüssigkeit unter Verwendung einer Vorrichtung mit einer Halterung zur Befestigung des Kristalls und einem Mikrodosiersystem gelöst, das im Verhältnis zur Halterung so angeordnet ist, daß damit Mikro-Tropfen einer Flüssigkeit, die bspw. Lösungsmittel und mindestens einen Ligandentyp aufweist, auf den in der Halterung befestigten Kristall aufgebracht werden können.

Durch das Auftropfen von Mikro-Tropfen mit der Vorrichtung läßt sich eine wesentlich schonendere Behandlung von Kristallen und insbesondere Proteinkristallen mit bestimmten aufzubringenden Substanzen, die in einer Lösung enthalten sind, erreichen. Diese Substanzen können bei Proteinkristallen Liganden, bspw. Inhibitoren, Substrate oder Reaktanden, sein. Bei den Liganden wird es sich bei Proteinkristallen typischerweise um Agonisten, Substrate oder Antagonisten der kristallisierten Proteine handeln. Weiterhin wird ein System zur Verfügung gestellt, das es erlaubt, einen Kristall, insbesondere einen Proteinkristall, unabhängig von der Umgebung in einer Mutterlösung, wie in allen Experimenten nach dem Stand der Technik mit einem Liganden zu komplexieren. Auf diese erfindungsgemäße Weise können Proteinkristalle auch in solchen Fällen, die mit den Verfahren nach dem Stand der Technik nicht mit Liganden komplexierbar sind, dennoch eine Komplexbildung eingehen. Die Ursache für die Überlegenheit des erfindungsgemäßen Verfahrens, das einen frei montierten Kristall und die Bereitstellung eines Mikro(Piko)tropfens durch Verwendung einer entsprechenden Vorrichtung voraussetzt, ist die Verschiebung des Gleichgewichts der Reaktion zwischen Ligand und kristallisiertem Protein zum komplexierten Protein. Dies wiederum hängt mit der Reduktion der apparenten Dissoziationskonstante K_{D} zusammen, da die Konzentration der freien Komponenten durch die Isolation der Proteinkristalls von der den Kristall (nach dem Stand der Technik) umgebenden Mutterlauge erheblich eingeschränkt ist. Diese Reduktion der K_{D} erlaubt es auch dann Komplexe zu erhalten, wenn die Bindungskonstante des Liganden an das kristallisierte Protein eigentlich gering ist oder der Ligand nur schwach löslich ist und daher Verfahren nach dem Stand der Technik (Kristall in Mutterlösung) keine oder nur eine geringfügige Komplexierung (die für röntgenkristallographische Folgeexperimente nicht ausreichend ist) ergeben.

Darüber hinaus ist es von erheblicher Bedeutung, bei der Komplexbildung nicht nur die erfindungsgemäß vorteilhafte Verschiebung des Gleichgewichts der Komplexierungsreaktion zu betrachten, sondern auch die durch das System mit frei montiertem Kristall vorteilhafte Kinetik der Komplexbildung, insbesondere bei schwach löslichen Liganden. Der erfindungsgemäß frei montierte Kristall (ohne die Umgebung einer Mutterlösung) hat eine größere Stabilität als der in der Mutterlösung nach dem Stand der Technik "gesoakte" Proteinkristall. Diese größere Stabilität kann genutzt werden, um bspw. die Komplexierung des Liganden, mit dem besonders bevorzugten Ziel mindestens 90%iger, vorzugsweise mindestens 95%iger Absättigung der im Kristall für den Liganden enthaltenen Bindungsplätze, durch die Verwendung von Verfahren zu erzwingen, denen ein Proteinkristall im Falle des "Soakings" oder der Kokristallisation nach dem Stand der Technik nicht zugänglich wären. Insbesondere vorteilhaft in diesem kinetischen Zusammenhang ist die Verwendung von auf Temperaturen oberhalb von 20°C erwärmter Ligandenlösung, die als Pikotropfen auf den frei montierten Kristall aufgetragen wird. Diese Erwärmung kann bspw. mindestens 30°C, vorzugsweise mindestens 40°C, noch stärker bevorzugt mindestens 50°C betragen. Auch eine Erwärmung bis zu 75°C ist möglich. Darüber hinaus oder in Kombination mit der Erwärmung der Ligandenlösung kann diese auf den Kristall, bspw. direkt aufgespritzte, als Pikotropfen aufgetragene Ligandenlösung auch organische Lösungsmittel enthalten oder aus diesen bestehen. Sofern das organische Lösungsmittel mit Wasser löslich ist (bspw. DMSO oder TFE) kann dieses zu mindestens 20 Vol-%, vorzugsweise zu mindestens 40 Vol.-% und noch stärker bevorzugt zu mindestens 50 Vol.-% in einem Wasser/organisches Lösungsmittel-Gemisch enthalten sein. Auch kann der Ligand in einem reinen organischen Lösungsmittel oder in einem Gemisch verschiedener organischer Lösungsmittel gelöst und als Mikrottopfen auf den frei montierten Kristall (sie hierzu im folgenden) aufgetragen werden. Der Einsatz organischer Lösungsmittel, der wiederum nur durch die erfindungsgemäße Verwendung eines frei montierten Kristalls und eines Mikrotropfens möglich wird, ist insbesondere dann bevorzugt, wenn die Liganden in wässriger Lösung nur schwer oder unlöslich sind. Schließlich kann der frei montierte Kristall auch einem Verdampferstrom ausgesetzt sein, wobei über einen Verdampfer organisches Lösungsmittel oder ein organische Lösungsmittelgemisch verdampft wird. Auf diese Weise wird das organische Lösungsmittel, bspw. DMSO oder Chlorkohlenwasserstoff, auf/im Kristall angereichert und dadurch die Löslichkeit des in Wasser schwer löslichen Liganden erhöht.

Gemäß einer Ausgestaltung ist in der in der Erfindung verwendeten Vorrichtung zum Behandeln eines Kristalls mit mindestens einer Substanz die Kristallhalterung so ausgebildet, daß durch die Halterung ein Gasstrom geführt werden kann, der auf den in der Halterung befestigten Kristall gerichtet ist. Dadurch kann der Kristall während der Behandlung durch die Mikro-Tropfen in einer definierten Umgebung gehalten werden.

Falls es sich bei dem Kristall um einen Proteinkristall handelt und die Substanz in der aufzutropfenden Flüssigkeit aus gelösten Liganden, bspw. Inhibitoren, besteht, die in die Kristallstruktur des Proteinkristalls eingebracht werden sollen, so kann dem Gasstrom auch gemäß einer weiteren vorteilhaften Ausführungsform ein Lösungsvermittler (s. obige Ausführungen) beigemischt werden, der insbesondere bei schwerlöslichen Liganden die Diffusion durch den Proteinkristall bzw. die Bindung an die kristallisierten Proteine wesentlich erleichtern kann.

Es ist ferner besonders vorteilhaft, daß die in der Erfindung verwendete Vorrichtung auch auf einem Goniometerkopf im Röntgenstrahl oder in einem Synchrotron befestigt werden kann, so daß der zeitliche Ablauf der Veränderung der kristallisierten Proteinstruktur, z.B. infolge der Ligandenbindung während des Auftropfens der Mikro-Tropfen, im Meßgerät beobachtet werden kann.

Die Aufgabe der Erfindung wird durch ein Verfahren zum Behandeln eines Kristalls entsprechend Anspruch 1 gelöst.

Weitere vorteilhafte Ausführungen des erfindungsgemäßen Verfahrens ergeben sich aus den abhängigen Ansprüchen.

Bevorzugte Ausführungsformen der vorliegenden Erfindung werden nachfolgend unter Bezug auf die beiliegende Zeichnung näher erläutert. Es zeigen
Fig. 1 eine teilweise im Schnitt dargestellte Ansicht einer Ausführungsform einer in der Erfindung verwendeten Vorrichtung zum Behandeln eines Kristalls mit einer Lösung,
Fig. 2 ein Gehäuse eines Steuergeräts zur Steuerung eines bei einer Ausführungsform der in der Erfindung verwendeten Vorrichtung verwendeten Mikrodosiersystems,
Fig. 3 zeigt ein Flüssigkeitszufuhrsystem für ein Mikrodosiersystem, das bei einer Ausführungsform der in der Erfindung verwendeten Vorrichtung verwendet werden kann.

Die Erfindung wird im folgenden am Beispiel der Behandlung von Proteinkristallen beschrieben, sie kann aber auch in analoger Weise bei der Behandlung von anderen Kristallen eingesetzt werden.

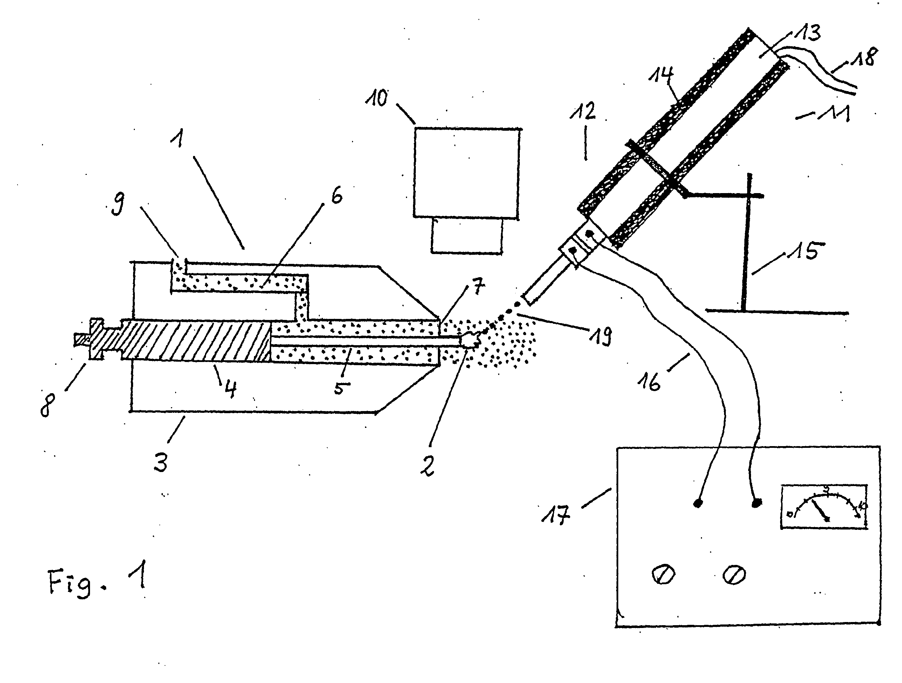

Fig. 1 zeigt eine erste Ausführungsform einer in der Erfindung verwendeten Vorrichtung zum Behandeln eines Kristalls. Dabei ist links in der Fig. 1 eine Halterung 1 dargestellt, die dazu dient, einen Proteinkristall 2 zu befestigen. Die in der Fig. 1 dargestellte Halterung, die in ihrer generischen Art auch als "free mounting system" (frei montiert) bezeichnet wird, ist bereits aus dem Stand der Technik bekannt und z.B. in der Deutschen Patentschrift DE 198 42 797 C1 beschrieben worden. Diese Druckschrift wird insoweit vollumfänglich in die Offenbarung der vorliegenden Anmeldung einbezogen. Im Sinne der vorliegenden Anmeldung ist ein frei montierter Kristall ein Kristall, der sich nicht in einer Flüssigkeit befindet, wie es etwa nach dem im Stand der Technik bekannten "Soaking" üblich ist.

Die Halterung 1, die in der Fig. 1 in einer Schnittansicht von der Seite dargestellt ist, besteht im wesentlichen aus einem Trägerblock 3, der ein einschiebbares Einsatzteil 4 aufweist, das in eine Öffnung des Trägerblocks 3 eingeschoben werden kann. Am Einsatzteil ist eine Haltekapillare 5 angebracht, an deren freiem Auflageende der Proteinkristall 2 gehalten wird. Die Haltekapillare besteht vorzugsweise aus einer Mikropipette, in der über eine in der Fig. 1 nicht dargestellte und mit dem anderen Ende der Mikropipette verbundene Pumpvorrichtung ein Unterdruck erzeugt wird, der dazu dient, den Proteinkristall 2 an dem freien Auflageende zu halten. Das linke Ende 8 des Einsatzteils ist so ausgebildet, daß daran die Halterung 1 an einem Goniometerkopf einer Röntgen- oder Synchrotronbestrahlungsanlage befestigt werden kann.

In einer Röntgen- oder Synchrotronbestrahlungsanlage kann die Beugung von Röntgenstrahlen beim Durchgang durch das Kristallgitter des Proteinkristalls ausgenutzt werden, um aus dem Beugungsbild auf die räumliche Anordnung der Atome und Moleküle in dem kristallisierten Protein zu schließen bzw. die Struktur durch mathematische Operationen zu errechnen. Die erforderlichen Röntgenstrahlen können z.B. durch Beschuß von Kupfer oder anderen Materialien mit Elektronen erzeugt werden (bspw. CuKα-Strahlung). Alternativ kann die Röntgenstrahlung auch in einem Synchrotron, d.h. einem Teilchenbeschleuniger, erzeugt werden, bei dem die Röntgenstrahlung von auf Kreisbahnen beschleunigten Elektronen emittiert wird. Das Synchrotron besitzt trotz des größeren apparativen Aufwands eine Reihe von Vorteilen gegenüber der herkömmlichen Erzeugung von Röntgenstrahlung durch Elektronenbeschuß von Metallen. So besitzen die durch Synchrotrone erzeugten Röntgenstrahlen eine höhere Intensität und können in verschiedenen Wellenlängen gewählt werden. Auch besteht auf diese Weise die Möglichkeit, "weißes" Röntgenlicht einzusetzen und damit den Kristall mit Röntgenblitzen, die Röntgenstrahlen aller Wellenlängen aufweisen, zu beschießen. Darüber hinaus lassen sich die Messungen mit dem Synchrotron wesentlich schneller als mit herkömmlichen Röntgenbestrahlungsanlagen durchführen.

In die Halterung 1 ist ferner ein Gaskanal 6 integriert, dessen Mündungsende 7 auf das freie Auflageende der Haltekapillare 5 gerichtet ist, an dem der Proteinkristall 2 befestigt ist. Dabei wird der am Auflageende angebrachte Proteinkristall 2 vollständig vom Gasstrom aus dem Gaskanal 6 umschlossen, so daß eine definierte Gasatmosphäre um den Proteinkristall herum erzeugt werden kann. Der Gaskanal 6 ist an seinem in der Fig. 1 als offen dargestellten Ende mit einem Gaserzeugungsmittel und einem Gasmischmittel verbunden, mit dem die Zusammensetzung des Gasstroms variabel eingestellt werden kann. Falls das um den Proteinkristall herum befindliche Gas Luft ist, kann das Gasmischmittel z.B. dazu dienen, die Luftfeuchtigkeit auf einen vorherbestimmten optimalen Wert einzuregeln. Es kann darüber hinaus auch ein Temperatureinstellmittel vorgesehen sein, mit dem die Temperatur des Gasstroms gemessen und auf einen bestimmten vorgebbaren Wert eingeregelt werden kann. Auch können andere gasförmige Substanzen dem Gasstrom beigemischt werden, so dass bspw. der Stickstoff- oder Sauerstoffgehalt der Luft modifiziert, bspw. erhöht, werden kann.

In der Deutschen Patentanmeldung Nr. 10232172.8-52 mit dem Titel "Vorrichtung und Verfahren zur Erzeugung einer definierten Umgebung für partikelförmige Proben" ist bereits eine Vorrichtung und ein Verfahren beschrieben, mit dem sich eine hochgenaue und langzeitstabile Feuchteeinstellung eines durch die oben beschriebene Halterung geführten feuchten Gasstroms am Ort des partikelförmigen Kristalls erreichen läßt. Diese Druckschrift wird daher insoweit ebenfalls vollumfänglich in die Offenbarung der vorliegenden Anmeldung einbezogen.

Über dem Kristall ist ein Mikroskop mit Videosystem 10 angebracht, mit dem der Proteinkristall während der Behandlung mit der Substanz beobachtet werden kann. Ggf. kann infolge der Beobachtung über das Videosystem der Behandlungsmodus modifiziert oder auch die Behandlung eingestellt werden.

Die in der Fig. 1 dargestellte in der Erfindung verwendete Vorrichtung zum Behandeln eines Kristalls mit einer Substanz umfaßt darüber hinaus ein Mikrodosiersystem 11, das rechts in der Fig. 1 in einer Seitenansicht im Schnitt dargestellt ist

Das Mikrodosiersystem 11 umfaßt eine sogenannte Piezopipette 12, die in einem Stativ 15 gehalten wird und so auf den Proteinkristall 2 ausgerichtet ist, daß dieser mittels der Piezopipette mit Tropfen beschossen werden kann. Die Piezopipette ist in der Fig. 1 aus Gründen der Anschaulichkeit in einem vergrößerten Maßstab im Verhältnis zur Halterung 1 dargestellt. Die Piezopipette ist so angeordnet, daß die Spitze der Piezopipette einen Abstand von typischerweise 3 mm zu dem Proteinkristall aufweist. Vorzugsweise liegt dieser Abstand in einem Bereich von 1 - 5 mm, kann jedoch unter besonderen Umständen auch größer oder kleiner gewählt werden.

Die Piezopipette 12 besteht aus einer Glaskapillare 13, die z.B. aus Borosilicatglas bestehen kann. Die Durchmesser der Öffnung der Glaskapillare ist einer der Faktoren, die die Größe der von der Piezopipette abgegebenen Mikro-Tropfen beeinflussen und kann z.B. in einem Bereich zwischen 5 und 50 Mikrometer liegen. Die Glaskapillare 13 ist von einem piezoelektrischen Element 14 umschlossen, das aus einem Material besteht, das einen piezoelektrischen Effekt zeigt Es kann sich bei diesem Material z.B. um einen Piezokristall handeln. Das piezoelektrische Element 14 ist darüber hinaus über zwei Kabel 16 mit einem Steuergerät 17 elektrisch verbunden, mit dem eine Spannung an das piezoelektrische Element 14 angelegt werden kann. Wird ein Spannungspuls durch das Steuergerät 17 an das piezoelektrische Element 14 angelegt, so wird das piezoelektrische Element 14 und mit diesem auch die Glaskapillare 13 kontrahiert und ein Tropfen aus der Öffnung der Piezopipette herausgeschossen. Über das Steuergerät 17 können unterschiedlich geformte Spannungspulse an die Piezopipette angelegt werden können, deren Formen die Form und Größe der Mikto-Tropfen und deren Frequenz die Frequenz der Mikro-Tropfen beeinflussen.

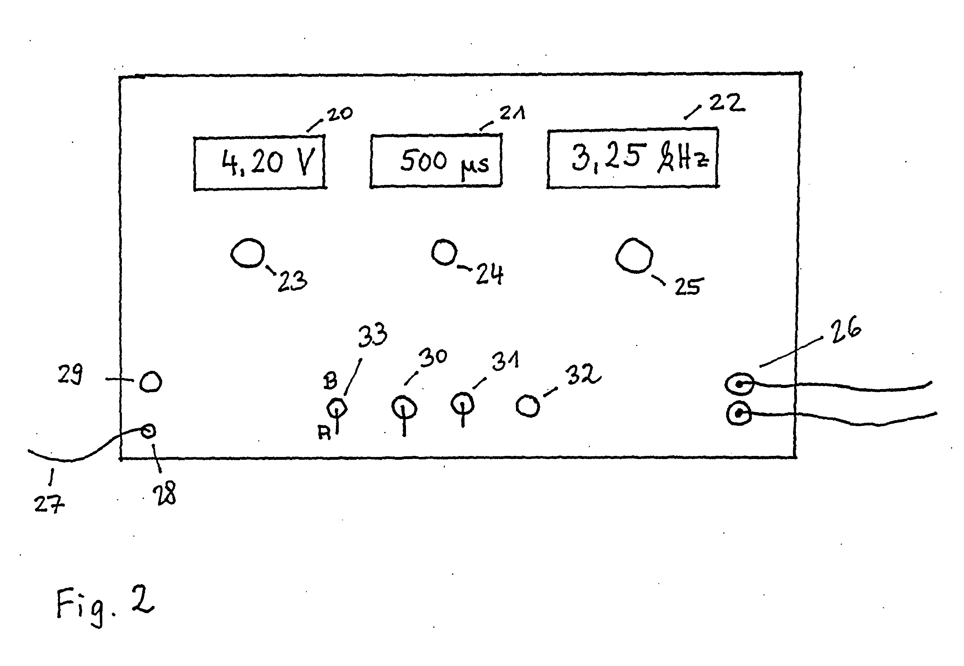

In der Fig. 2 ist ein Gehäuse eines möglichen Steuergeräts zur Steuerung der Piezopipette dargestellt, wobei die einzelnen Steuerungsmöglichkeiten anhand der in der Fig. 2 dargestellten Schalter und Steuerelemente des Steuergeräts erläutert werden sollen. Das Steuergerät weist zunächst drei verschiedene LCD-Anzeigen 20, 21 und 22 auf. Auf der ersten LCD-Anzeige 20 wird der aktuelle Wert für den Spannungspegel der Impulsausgangsspannung für das Piezopipettensteuersignal angezeigt. Dieser Wert läßt sich über einen Drehregler 23 variabel einstellen. Auch die Impulsweite des Pipettenansteuersignals, die auf der zweiten LCD-Anzeige 21 in Mikrosekunden angezeigt wird, läßt sich mittels eines zweiten Drehreglers 24 einstellen. Schließlich ist ein dritter Drehregler 25 vorgesehen, um die Frequenz der an die Piezopipette angelegten Spannungsimpulse einzustellen, die auf der dritten LCD-Anzeige 22 angezeigt wird. Diese Frequenz, die bis zu einige kHz betragen kann (z.B. 2 kHz) entspricht der Frequenz, mit der die Mikro-Tropfen aus der Piezopipette auf den Kristall geschleudert werden. Der Einstellbereich der Frequenz kann z.B. in einem Bereich zwischen 1 Hz und 6 kHz liegen. Die Höhe der Impulsausgangsspannung und die Weite der Spannungsimpulse müssen zunächst so eingestellt werden, daß es überhaupt zu einer Tropfenerzeugung mit der Piezopipette kommt. Darauf wird die Frequenz gewählt, die für den jeweiligen Kristallbehandlungsprozeß ideal ist. Die Frequenz kann natürlich auch während des Kristallbehandlungsprozesses laufend variiert werden.

Das Steuergerät weist ferner zwei Eingänge 26 auf, an denen die beiden Verbindungskabel der Piezopipette angeschlossen werden. Ferner sind ein Netzkabel 27 sowie ein Netzanschluß 28 zur Stromversorgung des Steuergeräts vorgesehen. Über den weiteren Signaleingang 29 können von anderen elektrischen Geräten vorgegebene Spannungsimpulsfolgen angelegt werden, um die Mikro-Tropfenerzeugung auszulösen und die Mikro-Tropfenfolge und -form von außen zu steuern. Das kann z.B. sinnvoll sein, wenn es ein zentrales Steuergerät gibt, das sowohl die Tropfenerzeugung als auch andere Parameter der Kristallbehandlung wie den über die Kristallhalterung zugeführten Gasstrom, die Zusammensetzung des Gasstroms (z.B. seinen Feuchtegehalt), die Temperatur des Gasstroms, eine angeschlossene Röntgenbestrahlungsanlage etc. steuert und die verschiedenen Steuerungsparameter in einer vorherbestimmten Weise zueinander synchronisiert.

Der Schalter 30 ist dazu vorgesehen, den Piezopipettenbetrieb ein- und auszuschalten. Über den weiteren Schalter 31 kann zwischen Einzelspannungsimpulsbetrieb und kontinuierlichem Spannungsimpulsbetrieb umgeschaltet werden, d.h. zwischen Einzeltropfenerzeugung und kontinuierlicher Tropfenerzeugung. Für die Einzeltropfenerzeugung kann ferner ein Taster 32 vorgesehen sein, über den einzelne Spannungsimpulse an die Piezopipette angelegt werden können, wenn es gewünscht ist, einzelne Tropfen per Handbetrieb auf den Kristall zu schießen.

Der Schalter 33 dient schließlich dazu zwischen verschiedenen Impulsformen der an die Piezopipette 12 angelegten Spannungsimpulse variieren zu können. In der Schalterstellung A kann z.B. ein vorgegebener Standard-Rechteckspannungsimpuls mit vorherbestimmter Dauer und Höhe erzeugt werden, während in der Schalterstellung B ein Rechteckspannungsimpuls erzeugt werden kann, dessen Dauer und Höhe variabel eingestellt werden kann. Es ist natürlich bei anderen Ausführungen auch denkbar, daß Spannungsimpulse angelegt werden, die von der Rechteckform abweichen. Die Impulsform der Spannungsimpulse wird nun so gewählt, daß eine optimale Tropfenerzeugung in Hinblick auf den zu behandelnden Kristall gewährleistet ist.

Verschiedene Größen der Mikro-Tropfen, die z.B. für verschiedene Kristallgrößen geeignet sein können, können über die Variation der Spannungspulsweiten und Spannungspulshöhen eingestellt werden, die die an die Piezopipette angelegten Spannungen aufweisen.

Die Glaskapillare 13 der Piezopipette 12 ist typischerweise über eine Zuleitung 18 mit einem in der Figur 1 nicht dargestellten Vorratsgefäß verbunden, das die Lösung enthält, die auf den Proteinkristall getropft werden soll. Diese Lösung enthält die Substanz oder die Substanzen, mit der bzw. denen der Proteinkristall behandelt werden soll. Die Oberkante des Flüssigkeitsspiegels der sich im Vorratsgefäß befindenden Flüssigkeit sollte dabei etwas höher als die Unterkante der Pipettendüse eingestellt werden. Alternativ dazu kann die Flüssigkeit bei einer Ausführungsform ohne Vorratsgefäß aber auch direkt über die Auslaßöffnung der Piezopipette in die Piezopipette gesaugt werden, um sie dann später wieder abgeben zu können. Es kann auch eine Temperiervorrichtung um das Vorratsgefäß herum angeordnet sein, um die in dem Vorratsgefäß sich befindende Flüssigkeit auf eine gewünschte Temperatur zu bringen. Gemäß einer Ausführungsform kann vor dem Aufbringen der Lösung auf den Kristall der pH-Wert und/oder die Ionenstärke (bzw. spezifische Salzkonzentrationen) der Lösung gemäß den im Stand der Technik bekannten Verfahren auf einen gewünschten Wert eingestellt werden.

Unter Mikro-Tropfen im Sinne der vorliegenden Erfindung sollen Tropfen zu verstehen sein, deren Volumen kleiner als 1 nl ist, wobei das Volumen der Mikro-Tropfen vorzugsweise zwischen 1 nl (nanoliter) und 1 pl (picoliter), noch weiter bevorzugt zwischen 100 pl und 20 pl und noch stärker bevorzugt zwischen 20 pl und 4 pl liegt. Aus diesen Größen lassen sich über die Volumensformel die entsprechenden geeigneten Durchmesser der Tropfen errechnen, wenn man näherungsweise davon ausgeht, daß die Tropfen kugelförmig sind. Die gewünschte Tropfengröße kann erfindungsgemäß eingestellt werden.

Die Mikro-Tropfen der auf den Kristall aufzubringenden Flüssigkeit sind dabei vorzugsweise kleiner als das Volumen des Kristalls ist. Ein typisches Kristallvolumen kann dabei z.B. in einer Großenördnung von 1 nl liegen.

Das Volumen der im speziellen Fall verwendeten Mikro-Tropfen wird in Abhängigkeit vom Volumen des Kristalls gewählt. Dabei betragen die Volumen der Mikrotropfen weniger als 50 %, z.B. 1 bis 20 %, des Kristallvolumens und vorzugsweise von 1, stärker bevorzugt von 5 bis 10 % des Kristallvolumens.

Die Tropfenerzeugung mittels einer Piezopipette ist nur ein Beispiel für eine Mikrodosiervorrichtung. Es können in der Erfindung auch andere Vorrichtungen verwendet werden, die in der Lage sind, Mikro-Tropfen zu erzeugen.

So kann z.B. auch ein Mikrodosiersystem verwendet werden, das eine Kapillare und ein in der Kapillare angeordnetes Mikroventil umfaßt Dabei wird die Flüssigkeit unter Druck aus einem Vorratsgefäß auf das Mikroventil gepreßt, das von einem Steuergerät elektrisch innerhalb eines kurzen Zeitintervalls geöffnet und danach wieder geschlossen wird, um die Tropfen zu erzeugen. Die Begrenzung der Tropfengröße ergibt sich hier durch die noch steuerbare Öffnungsdauer des Ventils.

Als Mikrodosiersystem kann bei einer anderen Ausführungsform auch ein Zerstäuber dienen. Ein Zerstäuber hat allerdings gegenüber den oben beschriebenen Lösungen den Nachteil, daß das Ausrichten der Tropfen auf den Kristall schwieriger ist. Daher wird vorteilhafter Weise dem Zerstäuber ein Mittel nachgeordnet, das die Orientierung der aus dem Zerstäuber erhaltenen Mikro-Tropfen auf den Kristall sicherstellt.

Es ist gemäß einer weiteren Ausführungsform einer in der Erfindung verwendeten Vorrichtung auch denkbar, daß die Mikrodosier-Vorrichtung aus einem "Loop", bspw. einer Schlaufe, besteht, mit dem einzelne Tropfen (oder nur ein Tropfen) auf den Kristall durch bspw. Abschütteln oder Abtropfenlassen von dem "Loop" aufgebracht werden. Es muß allerdings bei dieser Lösung der erfindungsgemäßen Aufgabe sichergestellt sein, daß die aufgebrachten Tropfenvolumina klein genug für die Proteinkristalle (im Sinn der voranstehend offenbarten Volumenverhältnisse von Kristall zu Tropfen) sind.

Auch alle weiteren technischen Möglichkeiten, Mikro-Tropfen entsprechender Größe zu erzeugen, sind Lösungen im Sinne der vorliegenden Erfindung.

Um die Frequenz der Auftragung der Mikrotropfen auf den Kristall variieren zu können, kann zwischen der in der Erfindung verwendeten Vorrichtung zur Tropfenerzeugung und dem Kristall eine Lochplatte, die bspw. mit einer gewissen Frequenz rotiert, angeordnet sein. Da - abhängig von der Vorrichtung zur Tropfenerzeugung - die Bereitstellung kleiner Mikrotropfen häufig eine höhere Tropfenfrequenz erforderlich macht, kann über die Zwischenschaltung einer Lochplatte, die nur jeden 2., 3. oder 4. oder weniger Tropfen auf den Kristall passieren lässt. Auch das auf den Kristall aufgetragene Volumen gesteuert werden.

Bei einer Ausführungsform des erfindungsgemäßen Verfahrens wird ein Proteinkristall zunächst an dem freien Auflagenende der Haltekapillare 2 befestigt. Anstelle der Haltekapillare 2 kann auch ein sogenannter "Loop", also eine Art Schlaufe, verwendet werden, in dem der Proteinkristall befestigt ist. Der Proteinkristall ist dabei frei von jeglicher Oberflächenlösung und damit zugänglich für Lösungen, die von außen direkt mittels des Mikrodosiersystems aufgebracht werden können. Durch die Halterung 1 wird nun eine Gasatmosphäre um den Proteinkristall 2 herum erzeugt, indem ein Gasstrom definierter Zusammensetzung und Temperatur durch den Gaskanal 6 der Halterung 1 geführt wird. Bei dem beschriebenen Verfahren wird es sich typischerweise um einen Luftstrom, ggf. unter Beimischung anderer gasförmiger Substanzen, mit einem geregelten Feuchtigkeitsgehalt (d.h. Wassergehalt) und einer geregelten Temperatur handeln.

In die Kristallstruktur des Proteinkristalls soll nun ein Inhibitor eingebracht werden, der Bestandteil einer Substanz ist, die der Lösung zugesetzt wurde, die sich in dem Vorratsgefäß befindet, das mit der Piezopipette verbunden ist. Es hat sich durch Experimente gezeigt, daß lokal auf die Oberfläche des Kristalls aufgebrachte Lösungen (wie bspw. DMSO) mit hoher Inhibitor-Konzentration den Kristall in der Regel nicht schädigen. Nun werden durch das Steuergerät 17 elektrische Spannungspulse an die Piezopipette 12 angelegt und Mikro-Tropfen mit der Inhibitor-Lösung auf den Proteinkristall 2 geschleudert. Durch das Aufspritzen einzelner Mikro-Tropfen bleibt der den Proteinkristall umströmende Gasstrom praktisch unbeeinflußt, so daß der Proteinkristall in seiner stabilen definierten Umgebung verbleibt. Die Erhaltung einer stabilen Umgebung ist insbesondere für die relativ instabilen Proteinkristalle, die durch geringe Gitterkräfte zusammengehalten werden, wichtig, damit die Kristalle nicht zerstört werden, bevor sie z.B. einer röntgenkristallographischen Untersuchung unterzogen werden. Die Feuchtigkeit des den Kristall umgebenden Luftstroms kann nun im Zusammenspiel mit der Größe und Frequenz der über die Mikrodosiervorrichtung auf den Proteinkristall aufgebrachten Tropfen so eingestellt werden, daß der Kristall möglichst sein Volumen nur wenig ändert, indem ein Gleichgewicht zwischen Abdampfen von Flüssigkeit vom Kristall und Zuwachs an Flüssigkeit durch Auftropfen von Flüssigkeit mittels der Mikrodosiervorrichtung erreicht wird. Dadurch wird der Kristall nur minimal belastet und es kann ein schonendes Einbringen des/der Liganden über die lokal aufgetragenen Mikrotropfen erreicht werden. Dieser Vorgang der Einstellung der optimalen Luftfeuchtigkeit bzw. der optimalen Auftropffrequenz durch die Mikrodosiervorrichtung kann über ein Regelelement automatisch geregelt werden, daß entsprechende Änderungen der Feuchtigkeit des Luftstroms und/oder der Auftropffrequenz vornimmt, wenn sich das gemessene Volumen des Kristalls ändert. Ziel ist es dabei, das Volumen des Kristalls möglichst konstant zu halten, d.h. das Volumen typischerweise um nicht mehr als 40%, bevorzugt nicht mehr als 20%, besonders bevorzugt nicht mehr als 10% vom Ausgangsvolumen abweicht. Die Volumensänderung kann dabei über eine Flächenprojektion gemessen werden..

Während des Kristallbehandlungsprozesses kann der Kristall gemäß einer bevorzugten Ausführungsform der Erfindung auch mit gepulstem Licht bestrahlt werden, z.B. über ein Stroboskop, um mittels des Videosystems in regelmäßigen Abständen eine Vermessung des Volumens des Tropfens durchführen zu können.

Das erfindungsgemäße Verfahren erweist sich auch dann besonders vorteilhaft, wenn Liganden, bspw. Inhibitoren oder andere Stoffe, in einen Kristall eingebracht werden sollen, die selbst in einer wäßrigen Lösung nur schwer zu lösen sind. Tatsächlich erweist sich eine Reihe von Liganden als in wäßrigen Systemen ausgesprochen schwer löslich, so daß mit dem in der Beschreibungseinleitung beschriebenen klassischen "Soaking"-Verfahren diese Liganden/Inhibitoren nicht in den Kristall eingebracht werden können, da die Konzentration der Liganden/Inhibitoren in der wäßrigen Lösung zu gering ist. Wird nun mittels des Mikrodosiersystems eine wäßrige Lösung, in der diese Liganden und/oder Inhibitoren gelöst sind, auf den Kristall aufgetropft, so verdunstet das Wasser nach jedem Auftropfen vollständig, während der Ligand auf bzw. im Kristall verbleibt. Durch wiederholte Auftropfzyklen können so größere Mengen des (schwer löslichen) Liganden auf den Kristall aufgebracht werden. Der Ligand akkumuliert sich so allmählich auf bzw. im Kristall, bis eine ausreichende Menge des Liganden in den Kristall eingebracht ist und eine zufriedenstellende Ligand-Protein-Komplexbildung (also die Besetzung des Kristalls an den Bindungsstellen der kristallisierten Proteine ausreichend ist, eine Elektronendichte für den Liganden zu bestimmen) erreicht ist.

Der Vorteil bei diesem Verfahren liegt auch darin, daß die Proteinkristalle nicht mit einem weiteren Lösungsmittel versetzt werden müssen und so die Behandlung der empfindlichen Kristalle schonender wird. Außerdem besteht derart nicht die Gefahr, daß der Ligand aufgrund seiner geringen Löslichkeit auf dem Kristall bzw. in den Lösungsmittelkanälen präzipitiert. Bei diesem Verfahren kann die Menge an durch das Mikrodosiersystem aufzutropfender Lösung durch die Konzentration der Lösung sowie einer Abschätzung der Molarität des Proteins im Kristall berechnet werden. Ein weiterer Vorteil des Verfahrens besteht darin, daß man mit Wasser als dem einzigen Lösungsmittel für den Liganden im Vergleich zu anderen Lösungsmitteln oder Flüssigkeiten besonders kleine Tropfengrößen erzielen kann, was insbesondere bei kleinen Proteinkristallen wichtig ist, da erfindungsgemäß die Tropfengröße kleiner als die Größe des Kristalls sein sollte.

Die in der Erfindung verwendete Vorrichtung zum Behandeln eines Kristalls mit einem Substrat kann auch in eine Röntgenbestrahlungsanlage oder Synchrotronbestrahlungsanlage integriert sein, so daß es möglich wird, während der Behandlung des Proteinkristalls mit der Substanz Beugungsbilder des Kristalls aufzunehmen und so den Behandlungsprozeß, d.h. die sukzessive Besetzung der Bindungsstellen des Kristalls, "online" zu beobachten. Hierzu kann die Halterung 1 z.B. an einem Goniometer einer Röntgen- oder Synchtotron-Bestrahlungsanlage befestigt werden. Der Proteinkristall kann vor der röntgenkristallographischen Untersuchung auch eingefroren werden, was in der Regel unter Verwendung von flüssigem Stickstoff erfolgt (sogenannte Cryo-Kristallographie). Hierdurch werden bei röntgenkrtstallographischen Untersuchungen die Intensitäten der Reflexe des Beugungsbildes ermittelt und schließlich unter Verwendung der Phaseninformation, z.B. aus isomorpher Ersetzung oder MAD ("multiple anomalous scattering"), die Elektronendichte der Struktur ermittelt werden.

Selbstverständlich können auch andere physikalische, insbesondere spektroskopische, Messungen mit Hilfe der Vorrichtung an dem Kristall durchgeführt werden. So kann die Vorrichtung z.B. auch mit einer Anlage zur Aufnahme einer Absorptionsspektrums kombiniert werden, um das Absorptionsspektrum des Kristalls aufzunehmen.

Gemäß einer weiteren bevorzugten Ausführungsform des erfindungsgemäßen Verfahrens kann auch oder nur dem durch die Halterung 1 geführten Gasstrom ein Lösungsvermittler beigefügt werden, der sich für die in den Proteinkristall einzubringende Substanz eignet, also z.B. ein Lösungsvermittler für einen schwerlöslichen Liganden. Hierzu kann zusätzlich ein Verdampfer vorgesehen sein, um den Lösungsvermittler vor der Einleitung in den Gaskanal 9 der Halterung 1 zu verdampfen. Auch kann eine Vorrichtung vorgesehen sein, die dazu dient, die Konzentration des Lösungsvermittlers im Gasstrom variabel einzustellen und den erforderlichen Bedingungen anzupassen. So läßt sich eine im Gegensatz zum klassischen "Soaking"-Prozeß sehr schonende Zufuhr von Lösungsvermittler zu dem Proteinkristall erreichen. Während der Zufuhr des den Lösungsvermittler enthaltenden Gasstroms kann dann über die Piezopipette die Ligandenlösung auf den Proteinkristall in Mikro-Tropfenform aufgebracht werden. Insgesamt ergibt sich also erfindungsgemäß die Möglichkeit nur der als Mikrotropfen aufzubringenden Ligandenlösung Lösungsvermittler oder nur dem zugeführten Gasstrom beizumischen. Ggf. können beide Alternativen auch kombiniert werden, so dass sowohl im Mikrotropfen als auch im Gasstrom der Lösungsmittelvermittler (gleich oder verschieden) zugesetzt werden. Auf erfindungsgemäße Weise lassen sich damit Liganden an den Kristall binden, die mit klassischen "Soaking"-Prozessen nicht gebunden werden können. Zudem ist ein solches erfindungsgemäßes Verfahren im Vergleich zu bisherigen "Soaking"-Prozessen mit geringerem Zeitaufwand verbunden, da wegen der schonenderen Kristallbehandlung weniger Versuche unternommen werden müssen, um die Kristallbehandlung erfolgreich zum Abschluß zu bringen.

Das erfindungsgemäße Verfahren eignet sich aber nicht nur zum Einbringen von Liganden in Proteinkristalle. Es können auch eine Reihe von anderen Behandlungsmethoden mit anderen Lösungen an Proteinkristallen auf die erfindungsgemäße Art durchgeführt werden.

Auch kann die über die Mikro-Tropfen mittels des Mikrodosiersystems aufgebrachte Lösung mehrere verschiedene Substanzen enthalten, mit denen der Kristall behandelt werden soll. Es kann sich dabei zum Beispiel um mehrere Liganden, bspum w. mehrere Substrate oder um ein Substrat und um einen katalytisch wirkenden Liganden, handeln, die in einer Lösung gelöst sind, die mittels einer Piezopipette auf den Kristall aufgebracht werden soll.

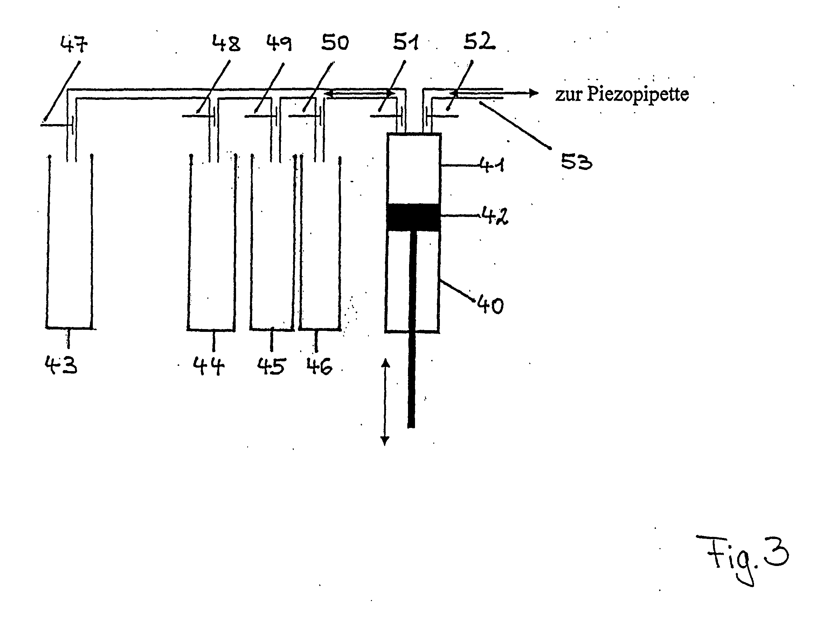

Gemäß einer weiteren Ausführungsform kann die Piezopipette auch mit einem speziellen Flüssigkeitzufuhrsystem versehen sein, mit dem es möglich ist, die Zufuhr verschiedener Flüssigkeiten in die Piezopipette zeitlich in gewünschter Weise zu steuern. In der Fig. 3 ist ein solches Flüssigkeitszufuhrsystem darstellt. Das in der Fig. 3 dargestellte Flüssigkeitszufuhrsystem umfaßt eine Präzisionsspritze 40, die aus einem Zylinder 41 besteht, in dem ein über einen (in der Fig. 3 nicht dargestellten) Motor angetriebener Kolben 42 hin- und herlaufen kann. Wenn der Kolben nach unten läuft, können verschiedene Flüssigkeiten aus den Flüssigkeitsbehältern 43, 44, 45, oder 46 in den Zylinder gesaugt werden, wenn eines der entsprechenden elektrisch ansteuerbaren Ventile 47, 48, 49 bzw. 50 geöffnet wird und zusätzlich das vor dem Zylinder liegende elektrisch steuerbare Ventil 51 geöffnet wird. Wird das Ventil 51 dann wieder geschlossen, das am Auslaß des Zylinders liegende elektrisch steuerbare Ventil 52 geöffnet und der Kolben 42 nach oben getrieben, so kann die angesaugte Flüssigkeit über die zur Piezopipette führende Flüssigkeitszufuhrleitung 53 zur Piezopipette gerührt werden, um dann schließlich in Tropfenform auf den Kristall gegeben werden zu können.

Die Behälter 45 und 46 können z.B. zwei verschiedene Lösungen mit verschiedenen Liganden enthalten, die mit dem Protein des zu betropfenden Kristall einen Komplex bilden sollen. Die Behandlung des Kristalls kann dabei z.B. so erfolgen, daß zunächst die Lösung 1 aus dem Behälter 45 und danach die Lösung 2 aus dem Behälter 46 auf den Kristall aufgetropft wird. Zwischen den beiden Lösungen kann eine Reinigungslösung durch die Leitungen gespült werden, die sich in dem Behälter 44 befindet. Der weitere Behälter 47 dient als Abfallbehälter, um Flüssigkeitsmengen aufzunehmen, die nicht mehr benötigt werden und aus dem Zufuhrsystem entfernt werden müssen. Durch geeignete zeitliche Ansteuerung der Ventile 47 - 52 und des Kolbens 42 können nun der Piezopipette die gewünschten Lösungen in der gewünschten Menge zugeführt werden.

Ein weiteres Beispiel zur Anwendung eines erfindungsgemäßen Verfahrens ist das sogenannte "Back-Soaking", bei dem bestimmte an die Kristallstruktur des Proteins bereits gebundene Substanzen durch andere Substanzen ausgetauscht werden, es wird also ein Cokristall erneut mit dem Ziel einer Substitution "gesoakt". So kann z.B. ein Ligand durch einen anderen Liganden, der sich in der Lösung befindet, die über Mikro-Tropfen auf den Proteinkristall aufgebracht wird, ersetzt werden.

Das erfindungsgemäße Verfahren kann ferner auch dazu verwendet werden, in sehr schonender Weise sogenannte "Cryo-Puffer" auf einen Proteinkristall (komplexiert oder nichtkomplexiert) aufzubringen. Viele Proteinkristalle müssen vor der röntgenkristallographischen Untersuchung aus Stabilitätsgründen eingefroren werden, was, wie oben beschrieben, in der Regel unter Verwendung von flüssigem Stickstoff durchgeführt wird. Die Cryo-Puffer werden während des Einfrierprozesses verwendet, um die Eisbildung zu verhindern, die zur Zerstörung des Proteinkristalls führen würde. Beispiele für Cryo-Puffer sind Glycerin oder 2-Methyl-2,4-pentanediol (MPD). Die Cryo-Puffer können in das Vorratsgefäß der Piezopipette gefüllt werden und dann in ähnlicher Weise wie die Ligandenlösung mit der Mikrodosiervorrichtung auf den Proteinkristall gespritzt werden.

Gemäß einer weiteren Ausführungsform des erfindungsgemäßen Verfahrens können auch mehrere Mikrodosiersysteme, z.B. mehrere Piezopipetten, verwendet werden, mit denen jeweils unterschiedliche oder auch identische Substanzen (bspw. an zwei verschiedenen, lokal abgegrenzten Bereichen des Kristalls) auf den Kristall aufgebracht werden. Eine solche Anordnung kann z.B. von Vorteil sein, wenn zwei verschiedene Liganden in eine Proteinkristallstruktur eingebracht werden sollen. Die Liganden werden dann in verschiedenen Lösungen gelöst, die in die beiden Flüssigkeitsvorratsbehälter zweier Piezopipetten gegeben werden. Über die beiden Piezopipetten werden dann die beiden Lösungen mit den verschiedenen Liganden in Mikro-Tropfenform auf den Proteinkristall aufgebracht. Dabei können über das mit einer Piezopipette jeweils verbundene Steuergerät, das die Tropfenerzeugung steuert, unterschiedliche Spannungsimpulse und Spannungspulsfolgen an die Piezopipetten angelegt werden, um so eine optimale Form und Frequenz der Mikro-Tropfen zu erreichen, die für den jeweiligen Liganden ideal ist

Die Verwendung von zwei Mikrodosiersystemen, mit denen getrennt zwei verschiedene Substanzen aufgebracht werden, die erst auf dem Kristall zusammentreffen, ist insbesondere auch dann vorteilhaft, wenn das kristallisierte Protein Katalysatorfunktion für die beiden Substanzen, die beide als Reaktanden im kristallisierten Protein gebunden werden, hat. Erfolgt das Aufspritzen der beiden Reaktanden separat durch zwei Mikrodosiersysteme während der Röntgenbestrahlung des Proteinkristalls, kann die Reaktion der Reaktanden unter katalytischem Einsatz der kristallisierten Proteine verfolgt werden. Eine Voraussetzung für eine derartige röntgenkristallographische Untersuchung ist natürlich die Stabilität des Kristalls, d.h., daß der Kristall seine Struktur nicht durch strukturelle Umlagerungen der kristallisierten Proteine verlieren darf, da er dadurch auch sein Diffraktionsvermögen verlieren würde.

Auch das sogenannte "Cryo-Soaking" läßt sich mit dem erfindungsgemäßen Verfahren in besonders vorteilhafter Weise durchführen. Das "Cryo-Soaking" ist durch die Kombination aus Ligandenzugabe und gleichzeitigem Einfrieren eines Proteinkristalls gekennzeichnet. So können z.B. Übergangszustände des Proteinkristalls eingefroren und dann röntgenkristallographisch untersucht werden. Auch hierzu kann ein System verwendet werden, das mit mehreren Mikrodosiersystemen arbeitet, wobei über das eine Mikrodosiersystem z.B. der oben beschriebene Cryo-Puffer und über das andere Mikrodosiersystem eine Lösung in Mikro-Tropfenform auf den Proteinkristall gegeben werden, die den in den Proteinkristall einzubringenden Liganden enthält.

Eine weitere Anwendung wäre etwa das Aufspritzen von Reaktanden, die in bestimmter Weise mit dem Proteinkristall reagieren.

Es ist gemäß einer weiteren Ausführungsform des erfindungsgemäßen Verfahrens auch denkbar, daß dem durch die Halterung für den Kristall geführten Gasstrom gewisse Substanzen zugefügt werden, mit denen der Kristall behandelt werden soll. So könnte z.B. beim "Soaking" dem Gasstrom ein in die Proteinkristallstruktur einzubringender Ligand beigemengt werden, während mit dem Mikrodosiersystem Lösungsmittel für diesen Liganden aufgetropft wird oder eine Lösung aufgetropft, in der ein weiterer Ligand gelöst ist, der gleichzeitig mit dem über den Gasstrom zugeführten Liganden in die Kristallstruktur des Proteinkristalls eingebaut werden soll. Das Lösungsmittel für den Liganden kann natürlich zusätzlich auch über den Gasstrom dem Kristall zugeführt werden. Hierzu kann ein Verdampfer eingesetzt werden, um das Lösungsmittel vorher in die Gasphase zu überführen. Auch ein Ligand, der über den Gasstrom zugeführt werden soll, kann über den Verdampfer dem Gasstrom zugeführt werden. Auch beim oben beschriebenen Cryo-Soaking kann z.B. über den Gasstrom Lösungsmittel mit Ligand dem Proteinkristall zugeführt werden, während über das Mikrodosiersystem ein Cryo-Puffer auf den Mikrokristall aufgetropft wird.

In einer bevorzugten Ausführungsform des erfindungsgemäßen Verfahrens im Wege des Hochdurchsatzes auch zur Identifikation von Verbindungen genutzt werden, die Komplexbildungseigenschaften aufweisen. Hierzu sind erfindungsgemäße Verfahren geeignet, wobei ein Kristall, wie bspw. in der DE 198 42 797 C1 oder in Figur 1 gezeigt montiert wird und (a) ein potentieller Ligand nach einem erfindungsgemäßen Verfahren auf den Kristall aufgebracht wird, (b) in einem zeitlichen Abstand von variabler Länge Beugungsintensitäten gemessen werden, wobei mindestens eine Aufnahme, vorzugsweise 2 bis 10 Aufnahmen, zu jedem Zeitpunkt aufgenommen werden, und (c) diese im zeitlichen Abstand gemessenen Beugungsintensitäten, die typischerweise verschiedene Akkumulierungszustände des potentiellen Liganden auf dem Kristall widerspiegeln, miteinander in ihrer zeitlichen Abfolge verglichen werden. Hierbei ist es besonders bevorzugt, wenn der Kristall in einer Orientierung während aller Beugungsaufnahmen verbleibt. Auf diesem Weg wird es erfindungsgemäß möglich mit nur einem Kristall und einzelnen Röntgenaufnahmen (ohne einen vollständigen Datensatz aufnehmen zu müssen), die Komplexbildung nachzuweisen und damit die Testsubstanz als Liganden oder als nicht-bindend zu identifizieren. Mit zunehmender Komplexbildung nimmt nämlich die Korrelation zum völlig unbesetzten Ausgangszustand des Kristalls ab, weswegen (im zeitlichen Abstand wachsende) Intensitätsunterschiede des Reflexe die Komplexbildung indizieren. Ein derartiges Verfahren kann als Hochdurchsatzverfahren durchgeführt werden, da eine nichtbindende Substanz verworfen werden kann und mit einer anderen Substanz das Verfahren gemäß Schritten (a) bis (c) wiederholt werden kann. Innerhalb von wenigen Minuten kann erfindungsgemäß eine Testsubstanz als Ligand identifiziert oder als nichtbindend verworfen werden.

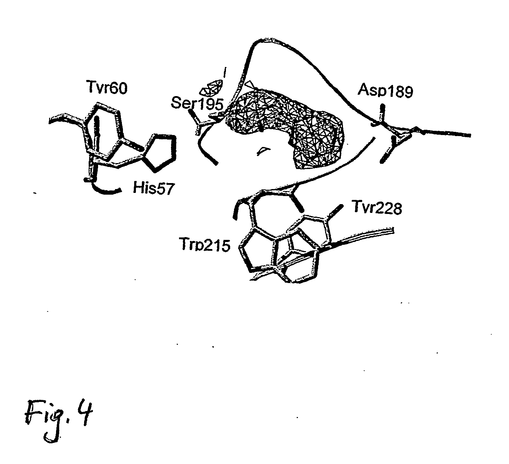

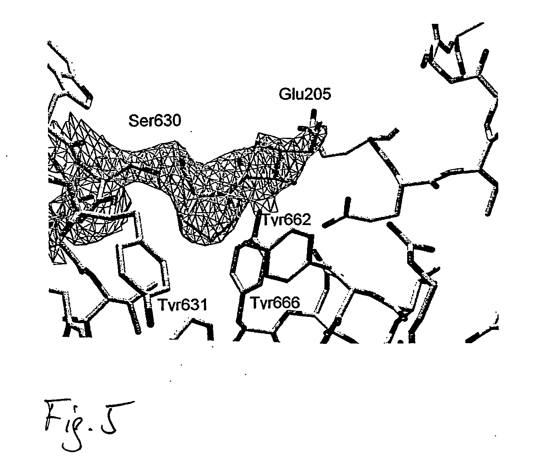

Die vorliegende Erfindung wird durch die Figuren 4 und 5 näher erläutert:
- **Figur 4:**: Der kovalent gebundene Inhibitor sowie einzelne Aminosäuren in der Umgebung des Aktiven Zentrums des Thrombins um Ser195 sind als stick-Modell dargestellt. Sauerstoffatome sind rot, Schwefelatome gelb, Stickstoffatome blau und Kohlenstoffatome grau dargestellt. Der Inhibitor ist zusätzlich von seiner 2Fₒ-F_{c}-Elektronendichte (kontouriert bei 1σ) überlagert. Der Inhibitor ist in seiner Elektronendichte eindeutig definiert.

In der experimentell bestimmten Elektronendichte ist deutlich die kovalente Bindung des PMSFs am Ser195 zu erkennen (Figur 4), die sich von der des ursprünglich im Kristall gebundenen Benzamidins signifikant unterscheidet. Damit wurde der Beweis geführt, daß der Ansatz des Auftropfens von Picoliter-Tropfen auf einen Proteinkristall unter Verwendung des Free Mounting Systems funktioniert

### Figur 5:

Das Spältprodukt Pro-Ile des Inhibitors Diprotin A sowie einzelne Aminosäuren in der Umgebung des Aktiven Zentrums der DPIV um Ser630 sind als stick-Modell dargestellt. Sauerstoffatome sind rot, Stickstoffatome blau und Kohlenstoffatome grau dargestellt. Der Inhibitor sowie Ser630, das kovalent an den Inhibitor gekoppelt ist, ist zusätzlich von seiner 2Fₒ-F_{c}-Elektronendichte (kontouriert bei 1σ) überlagert. Der Inhibitor ist in seiner Elektronendichte eindeutig definiert (Figur 5).

Vom eingesetzten Tripeptid mit der Sequenz Ile-Pro-Ile ist das C-terminale Isoleucin abgespalten, während das Dipeptid kovalent mit Ser630 weiterhin verknüpft ist und nicht abgespalten wird. Insofern verhält sich Diprotin A eher wie ein Suizid-Substrat und nicht wie ein Inhibitor.

Erfindungsgemäß ist weiterhin ein Verfahren Gegenstand der vorliegenden Patentanmeldung, bei dem die erfindungsgemäße Behandlung eines frei montierten Kristalls mit einer Vorrichtung zur Erzeugung von Mikrotropfen im Stapelbetrieb erfolgt, um mit einem hohen Durchsatz an zu komplexierenden Kristallen arbeiten zu können. Hierzu wird/werden erfindungsgemäß zunächst (a) der Kristall bzw. die Kristalle, vorzugsweise frei montiert, vorgehalten. Diese Vorhaltung der Kristalle bis zum nächsten Verfahrensschritt (b) kann bspw. durch Lagerung der Kristalle in tiefgekühltem Zustand oder aber, stärker bevorzugt, in einem abgeschlossenen Gefäß (z.B. Vials) im Dampfgleichgewicht mit der Kristallisationsflüssigkeit erfolgen, um die Unversehrtheit des Kristalls bzw. der Kristalle bis zum Verfahrensschritt (b) sicherzustellen. Im Verfahrensschritt (b) werden auf die die frei montierten Kristalle, wie erfindungsgemäß offenbart, Mikrotropfen einer bspw. einen Liganden enthaltenden Lösung aufgetragen, um den Kristall bspw. mit einem Liganden zu komplexieren. Nach der Komplexierung müssen die erfindungsgemäß behandelten Kristalle in einem Verfahrensschritt (c) gelagert werden, ehe in Verfahrenssschritt (d) die röntgenkristallographische Untersuchung erfolgen kann. Die Lagerung in Verfahrensschritt (c) wird typischerweise in tiefgekühltem Zustand, vorzugsweise in flüssigem Stickstoff, erfolgen. Die Durchführung der Verfahrensschritte (a) bzw. (c) kann bspw. in Probenwechslern erfolgen, wie sie in der Cryokristallographie eingesetzt werden, sog. "Autosampler". (z.B. vertrieben von Riken, Kouto, Japan oder X-Ray Research GmbH, Norderstedt, Deutschland). Hierbei werden die Proben au einem Probenträger angeordnet und dieser horizontal verschoben, um im Stapelbetrieb Proben durch eine Probenaufnahmevorrichtung aufnehmen zu können. Die Steuerung erfolgt automatisch. Gleichzeitig ist eine Vorrichtung zur Tiefkühlung vorgesehen.

Die vorliegende Erfindung wird durch die nachfolgenden Ausführungsbeispiele näher beschrieben.

### Ausführungsbeispiele

### 1. Ausführungsbeispiel

### Bindung von Phenylmethylsulfonyl-Flourid (PMSF) an humanes α-Thrombin

Ein Thrombin-Kristall wurde bei der zuvor ermittelten Startfeuchte von 93 % mit einem "Loop" auf dem Free Mounting System montiert. Überschüssiger Reservoirpuffer wurde aus dem "Loop" vorsichtig unter dem Mikroskop mit einem Filterpapierstreifen entfernt. Die Stabilität bzw. die Konstanz der Größe des Kristalls wurde mit Hilfe des Videosystems kontrolliert.

Darauf wurde die Piezopipette mit einer 100 mM Lösung von PMSF, einem Liganden (Inhibitor) von Thrombin, in Isopropanol befüllt. Es wurde hier eine hochkonzentrierte PMSF-Lösung in einem organischen Lösungsmittel verwendet.

Anschließend wurde der Kristall mit einzelnen Tropfen ("single shot" Modus) der Lösung "beschossen". Das insgesamt aufgetropfte Volumen entsprach ungefähr dem Volumen des Kristalls, also ca. 300 pl. Bei der sehr hohen eingesetzten PMSF-Konzentration entspricht dies ca. einem 3-4-fachen molaren Überschuß des Inhibitors. Dabei wurde nach jedem Auftropfen die Projektion der Fläche des Kristalls beobachtet und bis zum nächsten Auftropfen so lang gewartet, bis die Fläche wieder konstant blieb. Die einzelnen Parameter des Experiments sind Tabelle 1 zu entnehmen.

**Tabelle 1**

| | |
|---|---|
| Startfeuchte [% r.h.] | 93 |
| Größe der aufgebrachten Tropfen [pl] | ca. 30 |
| Anzahl der augebrachten Tropfen | 10 |
| Spannung [V] | 39,1 |
| Pulsweite [µs] | 450 |

Nach Abschluß des Auftropfens wurde der Kristall mit PFPE-Öl (Perfluoropolyether) benetzt und in flüssigem Stickstoff schockgefroren. Der röntgenkristallographische Datensatz wurde auf einer rotierenden Kupferanode aufgenommen. Die Prozessierung der Daten er-folgte mit den Programmen XDS und XSCALE, Verfeinerung und manueller Modellbau wurden mit den Programmen CNX und O durchgeführt. Die Statistiken von Datensammlung, Prozessierung und Verfeinerung sind in Tabelle 2 angegeben. Trotz des Auftropfens des reinen Lösungsmittels wurde die Diffraktionsqualität des Kristalls nicht beeinträchtigt, wie insbesondere anhand des Wertes von Rₘₑₐₛ zu erkennen ist.

**Tabelle 2**

| Inhibitor | PMSF |
|---|---|
| Strahlungsquelle | Rotierende Kupferanode |
| Wellenlänge [Å] | 1,5418 |
| Detektor | MAR Imageplate |
| Temperatur [K] | 100 |
| Raumgruppe | C2 |
| Zellparameter: | |
| a ≠ b ≠ c [Å] | 70,8; 72,8; 72,7 |
| α = γ = 90°; β | 99,77 |
| [°] | |
| Auflösung [Å] | 2,57 |
| Unabhängige | 10892 |
| Reflexe | |
| I/σ¹ | 7,3 (2,9) |
| Vollständigkeit¹ | 91,7 (94,5) |
| [%] | |
| Rₘₑₐₛ^{1,2} [%] | 11,8 (40,4) |
| R_{cryst}¹ [%] | 19,8 |
| R_{free}² [%] | 26,4 |

| | |
|---|---|
| ¹ Werte in Klammer gelten für die äußerste Auflösungsschale ² Rₘₑₐₛ = Σ \|I_{obs} - <I>\| / Σ <I> | |

Eine graphische Darstellung der Ergebnisse der röntgenkristallographischen Untersuchung ist Figur 4 zu entnehmen.

### 2. Ausführungsbeispiel

### Bindung von Diprotin A an Dipeptidyl-Peptidase IV (DPIV) aus Schwein

Die Kristalle der DPIV zeigen im nativen Zustand ein nur sehr eingeschränktes Streuverhalten. Erst durch Feuchte-Optimierung mit dem "Free Mounting System" lässt sich die erreichbare Auflösung der Kristalle aber entscheidend verbessern. Im optimierten Zustand bei reduzierter Feuchte zeichnen sich die Kristalle zudem durch eine erhöhte Stabilität aus und sind somit besser für "Soaking"-Experimente durch das Auftropfen geeignet.

Im vorliegenden Experiment wurde ein Kristall der DPIV bei der zuvor bestimmten Startfeuchte von 97 % mit dem "Free Mounting System" montiert. Zur Optimierung wurde die Feuchte in einem Gradient von 0,5 % Feuchteänderung pro 60 s auf 89 % r.h. abgesenkt. Die Transformation des Kristalls, die durch eine deutliche Verbesserung der Auflösung gekennzeichnet ist, beginnt bereits bei 94 % r.h. und erreicht bei 89 % r.h. ihr Maximum. Während des Auftropfens wurde daher die Feuchte konstant gehalten.

Als Ligand wurde eine Inhibitor-Lösung von wässriger Diprotin A Lösung (50 mM) verwendet. Bei Verwendung einer wässrigen Lösung sind im Vergleich zu organischen Lösungsmitteln kleinere Tropfengröße zu erreichen, wodurch der Kristall noch schonender behandelt wird. Während des Auftropfens der Lösung wurde permanent die Fläche des Kristalles beobachtet und erst dann der nächste Tropfen aufgetragen, wenn die Fläche sich nicht mehr änderte. Die Gesamt-Menge an aufgetropften Inhibitor entspricht einem ca. 10-fachen molaren Überschuß. Die einzelnen Parameter des Experiments sind Tabelle 3 zu entnehmen.

**Tabelle 3**

| | |
|---|---|
| Startfeuchte [% r.h.] | 97 |
| Feuchte-Gradient | 0,5 %/60s |
| Optimum der Feuchte [%] | 89 |
| Größe der aufgebrachten Tropfen [pl] | ca. 5-10 |
| Anzahl der aufgebrachten Tropfen | 80 |
| Spannung [V] | 39,2 |
| Pulsweite [µs] | 10 |

Nach Beendigung des Auftropfen wurde der Kristall unter Verwendung von PFPE-Öl (Perfluoropolyether) als *Cryo-Protectant* in flüssigem Stickstoff eingefroren. Die anschließende röntgenkristallographische Datensatzaufnahme erfolgte auf einer rotierende Kupferanode. Prozessiert wurden die Daten mit den Programmen XDS und XSCALE, Verfeinerung und manueller Modellbau wurden mit den Programmen CNX und O durchgeführt. Die Statistiken von Datensammlung, Prozessierung und Verfeinerung sind in Tabelle 4 angegeben.

**Tabelle 4**

| Inhibitor | Diprotin A |
|---|---|
| Strahlungsquelle | Rotierende Kupferanode |
| Wellenlänge [Å] | 1,5418 |
| Detektor | MAR Imageplate |
| Temperatur [K] | 100 |
| Raumgruppe | P1 |
| Zellparameter: | |
| a ≠ b ≠ c [Å] | 62,3; 118,5; 133,1 |
| α ≠ β ≠ γ [°] | 112,7; 94,9; 90,9 |
| Auflösung [Å] | 2,59 |
| Unabhängige | 103898 |
| Reflexe | |
| I/σ¹ | 14,0 (4,1) |
| Vollständigkeit¹ | 93,5 (89,4) |
| [%] | |
| Rₘₑₐₛ^{1,2} [%] | 6,1 (28,0) |
| R_{cryst}¹ [%] | 22,6 |
| R_{free}² [%] | 27,5 |

Die Ergebnisse der röntgenkristallographischen Untersuchung sind in Figur 5 dargestellt. Mit diesem Ausführungsbeispiel wird das große Potential erfindungsgemäßer Verfahren unter Verwendung von Microdosiersystemen (und Free Mounting) deutlich, da hier parallel ein Kristall optimiert und durch das Auftropfen ein Inhibitor gebunden werden konnte.

### 3. Ausführungsbeispiel

### Bindung von Pefabloc an humanes α-Thrombin durch langsames Akkumulieren

Eine weitere Möglichkeit, insbesondere schwerlösliche Liganden (Inhibitoren) an kristallisierte Proteine zu binden, besteht im langsamen Akkumulieren des Inhibitors, der im wässrigen System gelöst ist. Beim klassischen "Soaking" ist man stets darauf angewiesen, daß die Konzentration des Inhibitors in der Lösung nicht zu gering ist. Daher ist es häufig notwendig, dem Ansatz zur Erhöhung der Löslichkeit Lösungsmittel beizumischen, was die empfindlichen Proteinkristalle u.U. schädigt oder sogar zerstört.

Bei Durchführung eines erfindungsgemäßen Verfahrens wurde zur schonenderen Behandlung ganz auf Lösungsmittel verzichtet, da sich der Inhibitor auch bei nur geringer Löslichkeit durch häufiges Auftropfen im Kristall akkumuliert, wodurch die Wahrscheinlichkeit der Bindung wesentlich erhöht wird. Durch die Beobachtung des Kristalls mit dem Videosystem wurde gewährleistet, daß der Kristall nicht zu stark befeuchtet wurde. Ein neuer Tropfen wurde erst dann aufgebracht, wenn der vorherige Tropfen bereits verdunstet war. Aus den Angaben über die Löslichkeit des jeweiligen Inhibitors, der Tropfengröße und der Anzahl der Moleküle im Kristall konnte sogar die Anzahl der aufzubringenden Tropfen berechnet werden.

Als Inhibitor wurde Pefabloc SC (4-(2-Aminoethyl)-benzolsulfonyl-fluorid-hydrochlorid, Kᵢ: 6,5 µM) verwendet, der sich durch eine hohe Stabilität im wässrigen System auszeichnet.

Es wurde die Anzahl benötigter Tropfen für eine Stöchiometrie von Inhibitor zu Protein von 2:1 errechnet unter folgenden Randbedingungen: bei einem Kristall-Volumen von 300 pl und einer Dichte des Kristalls von ca. 1,3 g/ml ergab sich für die Masse des Kristalls = 390 ng. Unter Berücksichtigung des Molekulargewichts von Thrombin (ca. 40 kDa) ergab sich eine Konzentration des Thrombins im Kristall ca. 9,75 pmol/300 pl bzw. 32,5 mM. Da es das Ziel war, den Inhibitor im stöchiometrischen Verhältnis von 2:1 aufzubringen, um eine möglichst hohe Besetzung zu erreichen, betrug die benötigte Menge des Inhibitors: 19,5 pmol (9,75 pmol x 2).

Die Menge an aufzutropfender Inhibitor-Lösung hängt von der Konzentration des Inhibitors ab und wurde experimentell für drei Konzentrationen unter der Annehme einer Tropfengröße von 10 pl, wie sie bei Verwendung von Wasser realisierbar ist, gewählt, wie in Tabelle 5 dargestellt. Bei einer üblichen relativen Feuchte zwischen 90 und 100 % r.h. konnte regelmäßig 1 Tropfen pro sec auf den Kristall aufgebracht werden. Die Frequenz wurde aber deutlich erhöht, wenn bei reduzierter Feuchte im Hüllstrom, die zu einem Austrocknen der Kristalle führt, der Verlust an Feuchtigkeit durch ein schnelleres Auftropfen ausgeglichen und somit die rel. Feuchte am Kristall konstant gehalten werden mußte. Dadurch wurde eine Frequenz von 20 Tropfen pro Sekunde ermöglicht. Die sich daraus bei verschiedenen Inhibitorkonzentrationen ergebenden Zeiten sind ebenfalls in Tabelle 5 dargestellt.

**Tabelle 5**

| Inhibitor-Konzentration | Gesamtvolumen an aufzutropfender Lösung [µl] | Anzahl der Tropfen (Tropfenvolumen 10 pl) | Dauer des Experiments [h] bei 20 Tropfen/sec |
|---|---|---|---|
| 100 µM | 0,195 | 19.500 | 0,3 |
| 10 µM | 1,95 | 195.000 | 2,7 |
| 1 µM | 19,5 | 1.950.000 | 27,1 |

Beim Experiment mit humanen α-Thrombin und dem Inhibitor Pefabloc unter Verwendung einer Konzentration von 100 µM wurden die folgenden, aus Tabelle 6 entnehmbaren experimentellen Parameter gewählt. Durch die erhöhte Frequenz beim Auftropfen wurde die reduzierte Feuchte während des Experimentes ausgeglichen werden.

**Tabelle 6**

| | |
|---|---|
| Startfeuchte [% r.h.] | 93 |
| Feuchte beim Auftropfen [% r.h.] | ca. 80 |
| Inhibitor-Konz. [µM] | 100 |
| Größe des Proteinkristalls [pl] | 250 |
| Größe der aufzubringenden Tropfen [pl] | ca. 10 |
| Frequenz [s⁻¹] | 10 |
| Anzahl der aufzubringenden Tropfen | 17.000 |
| Spannung [V] | 41,0 |
| Pulsweite [µs] | 450 |

## Patentansprüche

1. Verfahren zum Behandeln eines Kristalls mit einer eine oder mehrere Substanzen enthaltende Lösung mit den folgenden Schritten:
- Befestigen des Kristalls in einer Halterung, wobei der in der Halterung befestigte Kristall nicht in eine flüssige Umgebung eingebettet ist ,
- Erzeugen eines Gasstroms definierter Zusammensetzung um den Kristall herum; und
- direktes Aufbringen von Mikrotropfen der Lösung auf den befestigten Kristall.

2. Verfahren nach Anspruch 1, bei dem der Gasstrom aus einem Luftstrom mit kontrollierter Luftfeuchtigkeit besteht oder der Gasstrom während des Auftropfens geregelt wird.

3. Verfahren nach Anspruch 2, bei dem die Luftfeuchtigkeit des Gasstroms und die Frequenz, mit der die Tropfen durch das Mikrodosiersystem auf den Kristall aufgetropft werden, während des Auftropfens so aufeinander abgestimmt werden, dass der Kristall möglichst wenig belastet wird, insbesondere das Volumen des Kristalls sich um nicht mehr als 20%, insbesondere um nicht mehr als 10% verändert.

4. Verfahren nach einem der Ansprüche 1 bis 3, bei dem der Gasstrom einen Lösungsvermittler in kontrollierter Konzentration für eine auf den Kristall aufzubringende Substanz umfasst.

5. Verfahren nach einem der Ansprüche 1 bis 4, bei dem das Volumen der Mikrotropfen kleiner als das Volumen des Kristalls ist, wobei vorzugsweise die Mikrotropfen der Lösung ein Volumen zwischen 1 nl und 100 pl, vorzugsweise zwischen 100 pl und 20 pl und noch bevorzugter zwischen 20 pl und 4 pl aufweisen.

6. Verfahren nach einem der Ansprüche 1 bis 3, bei dem der Kristall ein Proteinkristall ist.

7. Verfahren nach einem der Ansprüche 1 bis 6, wobei die Lösung auf eine Temperatur oberhalb von 20°C erwärmt ist, und vorzugsweise die eine oder mehreren Substanzen einen oder mehrere Liganden oder Inhibitoren enthalten.

8. Verfahren nach Anspruch 7, bei dem die Lösung aus Wasser oder einem organischen Lösungsmittel oder einem Gemisch aus organischen Lösungsmittel(n) und/oder Wasser besteht.

9. Verfahren nach Anspruch 5 oder 8, bei dem die eine oder mehreren Substanzen aus einem oder mehreren in Wasser nur schwer zu lösenden Inhibitoren oder Liganden bestehen.

10. Verfahren nach Anspruch 1, bei dem die Lösung einen Cryo-Puffer enthält.

11. Verfahren nach einem der Ansprüche 1 bis 10, bei dem der Gasstrom eine oder mehrere Substanzen enthält, die einen oder mehrere Liganden oder Inhibitoren enthalten.

12. Verfahren nach einem der Ansprüche 1 bis 11, bei dem der Kristall mit Hilfe eines Verdampfers mit Lösungsmittel, insbesondere organischem Lösungsmittel, bedampft wird.

13. Verfahren zur Ermittlung einer proteinkristallographischen Struktur, ggf. eines Komplexes eines Proteins und einer Substanz, wobei die Verfahrensschritte nach einem der Ansprüche 1 bis 12 durchgeführt werden und darüber hinaus der Kristall mit Röntgenstrahlung oder Synchrotronstrahlung bestrahlt wird, und das Beugungsbild des Kristalls aufgenommen wird.

14. Verfahren nach Anspruch 13, bei dem die Bestrahlung während der Behandlung des Kristalls mit der Lösung stattfindet.

15. Verfahren nach einem der Ansprüche 13 oder 14, bei dem darüber hinaus die Intensitäten der Reflexe des Beugungsbildes ermittelt werden.

16. Verfahren nach einem der Ansprüche 13 bis 15, bei dem darüber hinaus unter Verwendung der Phaseninformation, z.B. aus isomorpher Ersetzung oder MAD (multiple anomalous scattering), die Elektronendichte der Kristallstruktur ermittelt wird.

17. Verfahren zur Identifikation eines ein kristallisiertes Protein bindenden Liganden, bei dem (a) ein potentieller Ligand nach einem Verfahren nach einem der Ansprüche 1 bis 12 auf den Kristall aufgebracht wird, (b) in einem zeitlichen Abstand von variabler Länge Beugungsintensitäten gemessen werden und (c) diese im zeitlichen Abstand gemessenen Beugungsintensitäten entsprechend ihrer zeitlichen Abfolge miteinander verglichen werden.

18. Verfahren nach einem der Ansprüche 1 bis 17 zur röntgenkristallographischen Strukturaufklärung im Hochdurchsatz, bei dem die mit Mikrotropfen der Lösung behandelten Kristalle gelagert und röntgenkristallographisch untersucht werden.

## Claims

1. A method for treating a crystal with a solution containing one or more substance/s, the method having the following steps:
- fixing the crystal in a holder, wherein the crystal fixed in the holder is not surrounded by a liquid environment,
- generating a gas stream of defined composition around the crystal; and
- applying microdrops of the solution directly onto the fixed crystal.

2. The method according to claim 1, wherein the gas stream consists of an air stream of regulated air humidity or the gas stream is regulated during dripping-on.

3. The method according to claim 2, wherein the air humidity of the gas stream and the frequency, at which the drops are dripped onto the crystal by means of the micro dosage system, are coordinated during dripping-on in such a way, that the crystal is strained as little as possible, in particular that the volume of the crystal changes by no more than 20%, in particular by no more than 10%.

4. The method according to any one of claims 1 to 3, wherein the gas stream comprises a solubilizer of regulated concentration for a substance to be applied onto the crystal.

5. The method according to any one of claims 1 to 4, wherein the volume of the microdrops is smaller than the volume of the crystal, wherein preferably the microdrops of the solution have a volume between 1 nl and 100 pl, preferably between 100 pl and 20 pl, and even more preferably between 20 pl and 4 pl.

6. The method according to any one of claims 1 to 3, wherein the crystal is a protein crystal.

7. The method according to any one of claims 1 to 6, wherein the solution is heated to a temperature higher than 20°C, and preferably the one or more substance/s contain/s one or more ligand/s and/or inhibitor/s.

8. The method according to claim 7, wherein the solution contains water or an organic solvent or a mixture of organic solvent/s and/or water.

9. The method according to claim 5 or 8, wherein the one or more substance/s comprise/s one or more inhibitor/s or ligand/s, being only hardly soluble in water.

10. The method according to claim 1, wherein the solution comprises a cryo buffer.

11. The method according to any one of claims 1 to 10, wherein the gas stream contains one or more substance/s containing one or more ligand/s and/or inhibitor/s.

12. The method according to any one of claims 1 to 11, wherein the crystal is vapor-plated with solvent, in particular organic solvent, by means of an evaporator.

13. A method for determining a protein crystallographic structure, optionally a complex of a protein and a substance, wherein the method steps according to any one of claims 1 to 12 are conducted and furthermore the crystal is irradiated with X-ray or synchrotron radiation, and the diffraction image of the crystal is recorded.

14. The method according to claim 13, wherein the irradiation occurs during the treatment of the crystal with the solution.

15. The method according to any one of claims 13 or 14, wherein furthermore the intensities of the reflexes of the diffraction image are determined.

16. The method according to any one of claims 13 to 15, wherein furthermore the electron density of the crystal structure is determined by using the phase information, for example from isomorphic substitution or MAD (multiple anomalous scattering).

17. A method for identifying ligands binding a crystallized protein, wherein (a) a potential ligand is applied onto the crystal by means of a method according to any one of claims 1 to 12, (b) diffraction intensities are measured at time intervals of variable length, and (c) said diffraction intensities measured at time intervals are compared with respect to their chronological sequence.

18. The method according to any one of claims 1 to 17 for X-ray crystallographic structure determination at high throughput, wherein the crystals treated with microdrops of the solution are stored and are examined X-ray crystallographically.

## Revendications

1. Procédé de traiter un cristal avec une solution contenant une ou plusieurs substances, comprenant les étapes suivantes:
- fixer le cristal dans une fixation, le cristal fixé par la fixation n'étant pas entouré d'un environnement liquide,
- créer un courant de gaz avec une composition définie autour du cristal; et
- appliquer directement les microgouttes de la solution sur le cristal fixé.

2. Procédé selon la revendication 1, dans lequel le courant de gaz consiste en un courant d'air à humidité de l'air controlée ou bien le courant de gaz est réglé pendant l'application des gouttes.

3. Procédé selon la revendication 2, dans lequel la humidité de l'air du courant de gaz et la fréquence avec laquelle les gouttes sont appliqués par le système de microdosage sur le cristal sont accordées l'un sur l'autre de la manière, telle que le cristal sera chargé le moins possible, notamment le volume du cristal ne changera pas plus de 20 %, en particulier il ne changera pas plus de 10 %.

4. Procédé selon l'une des revendications 1 à 3, dans lequel le courant de gaz comprend un adjuvant de dissolution avec une concentration contrôlée pour une substance à appliquer sur le cristal.

5. Procédé selon l'une des revendications 1 à 4, dans lequel le volume des gouttes d'eau est plus petit que le volume du cristal, les microgouttes de la solution ayant de préférence un volume entre 1 nl et 100 pl, de préférence entre 100 pl et 20 pl et encore plus préfère entre 20 pl et 4 pl.

6. Procédé selon l'une des revendications 1 à 3, dans lequel le cristal est un cristal de protéine.

7. Procédé selon l'une des revendications 1 à 6, dans lequel la solution est échauffée à une température au-dessus de 20 °C et de préférence l'une ou les plusieurs substances contenant un ou plusieurs ligands ou inhibiteurs.

8. Procédé selon la revendication 7, dans lequel la solution se compose de l'eau ou un mélange de solvant(s) organique(s) et/ou de l'eau.

9. Procédé selon la revendication 5 ou 8, dans lequel l'une ou les plusieurs substances consistent en un ou plusieurs inhibiteurs ou ligands qui ne sont que difficile à dissoudre dans l'eau.

10. Procédé selon la revendication 1, dans lequel la solution contient un tampon cryo.

11. Procédé selon l'une des revendications 1 à 10, dans lequel le courant de gaz contient une ou plusieurs substances contentant un ou plusieurs ligands ou inhibiteurs.

12. Procédé selon l'une des revendications 1 à 11, dans lequel le cristal est vaporisé à l'aide d'un vaporisateur avec le solvant, particulièrement avec un solvant organique.

13. Procédé d'investigation d'une structure protéine-cristallographique et, le cas échéant, d'un complexe d'une protéine et d'une substance, dans lequel les étapes de procédure sont réalisées selon l'une des revendications 1 à 12 et, de plus, le cristal est irradié avec des rayons de Roentgen ou de synchrotron et l'image de diffraction du cristal est enregistrée.

14. Procédé selon la revendication 13, dans lequel l'irradiation a lieu pendant le traitement du cristal avec la solution.

15. Procédé selon l'une des revendications 13 ou 14, dans lequel en outre l'intensité des reflets de l'image de diffraction est investiguée.

16. Procédé selon l'une des revendications 13 à 15, dans lequel, en outre, en utilisant l'information de phase, par exemple dérivée de la substitution isomorphe ou de MAD (multiple anomalous scattering), la densité d'électrons est trouvée.

17. Procédé d'identification d'un ligand reliant à une protéine cristallisée, dans lequel (a) un ligand potentiel est appliqué sur le cristal par un procédé selon l'une des revendications 1 à 12, (b) des intensités de diffraction sont mesurées dans un intervalle de durée variable et (c) ces intensités de diffraction sont comparées ensembles selon leur séquence de temps.

18. Procédé selon l'une des revendications 1 à 17 pour la résolution de structure en haute capacité, dans lequel les cristaux traités avec des microgouttes de la solution sont stockés et investigués par cristallographie des rayons X.
